# EUROPÄISCHE PATENTSCHRIFT

(11) **EP 1 930 158 B1**
(45) Veröffentlichungstag und Bekanntmachung des Hinweises auf die Patenterteilung: **27.02.2013**
(21) Anmeldenummer: 07022642.8
(22) Anmeldetag: 22.11.2007
(51) Int. Cl.: B32B 38/00, B32B 38/04, A61F 13/15

(54) **Elastische Verbunde und ihre Herstellung**
Elastic compounds and their manufacture
Composites élastiques et leur fabrication

(30) Priorität: 05.12.2006 DE 102006057235
(43) Veröffentlichungstag der Anmeldung: 11.06.2008
(73) Patentinhaber: RKW SE, 67227 Frankenthal (DE)
(72) Erfinder: Waller, Paul, 83026 Rosenheim (DE); Bauer, Peter, 83512 Wasserburg (DE)
(74) Vertreter: Wagner, Jutta

(56) Entgegenhaltungen:
- EP-A- 0 546 792
- WO-A-2005/065932
- WO-A-2005/068165
- US-A1- 2003 198 797

## Beschreibung

Die vorliegende Erfindung betrifft elastische Verbunde aus mindestens einer Substratlage und mindestens einer Folienlage sowie deren Herstellung.

Elastische Verbunde bestehend aus einer elastischen Folienschicht und einseitig oder beidseitig aufgebrachten textilen Substraten (z.B. Vliesen und Gewirken) sind aus der Literatur bekannt. Üblicherweise werden diese elastischen Verbunde in Hygieneartikeln eingesetzt, wo durch elastische Materialien eine Erhöhung des Komforts gegeben ist. Bei Baby und Erwachsenenwindeln sind z.B. Verschlusssysteme, Bereiche im Bundbereich oder aber auch das gesamte Windelchassis elastisch gestaltet. Ein weiterer Anwendungsbereich ist Einwegbekleidung.

Elastische Verbunde aus Substrat und Folie sind in der Lage eine Vielzahl der an solche Artikel gestellten Forderungen zu erfüllen. Die Oberfläche wird durch eine oder mehrere Substratlagen sowohl in der Optik als auch in der Haptik an diejenige von Textilien angenähert. Die Substratoberflächen weisen eine faserige Struktur auf, die dem Aussehen von Textilien sehr ähnelt. Auch beim Anfassen ergibt sich das von Textilien gewohnte Gefühl.

Da im Allgemeinen die Substrate keine, oder nur eine geringe Elastizität aufweisen, muss für den bestimmungsgemäßen Gebrauch des Verbundes als elastischer Verbund das Substrat "gebrochen" werden. Dabei wird bisher der Verbund vorgedehnt, wobei die elastische Folie diesem Dehnprozess folgt, das Substrat jedoch nicht und das Substrat damit an diskreten Stellen zerstört wird. Diesen Vorgang bezeichnet man als "Aktivierung". Üblicherweise erfolgt die Aktivierung in Querrichtung mittels gerillten, ineinander greifenden Walzenpaaren. Nach der Aktivierung ist der Verbund elastisch.

Dieser allgemein bekannte Vorgang der Aktivierung zeigt in der Praxis jedoch folgende Nachteile:
- Die Aktivierung ist auf relativ einfache geometrische Strukturen, wie Linien und unterbrochene Linien beschränkt.
- Durch die Aktivierung des Verbundes wird die elastische Folie bereits vorgedehnt und unter Umständen dadurch geschädigt bzw. auf jeden Fall die ursprünglichen Eigenschaften verändert.
- Das Substrat bricht durch diese Art der Aktivierung in unregelmäßigen Linien.
- die Verbundhaftung kann beeinträchtigt werden.

Durch den Einsatz von elastischen Substraten (z.B. Vliesen die aus elastischen Thermoplasten hergestellt wurden) können diese negativen Einflüsse größtenteils eliminiert werden, da bei solchen Verbunden eine Aktivierung je nach Anwendungsfall nicht nötig ist. Diese elastischen Substrate sind jedoch sehr teuer und haben sich am Markt bislang nicht durchsetzen können.

Es bestand daher die Aufgabe, alternative elastische Verbunde und Verfahren zu ihrer Herstellung anzugeben.

Aus US 2003/198797 ist eine Schneidunterlage bekannt, die eine absorbierende Materiallage und eine schneidfeste, flüssigkeitsdurchlässige Oberfläche umfasst. Als Oberfläche eignet sich auch eine Folie, in welche mittels Vakuum, Nadeln, Wasserstrahlen, Laser etc. Öffnungen eingebracht sind. Die EP 546 792 beschreibt ein Laminat, welches sich als Verpackungsmaterial eignet. In einer Lage des Laminates werden vor dem Laminieren mittels einer Stanzeinrichtung, die auch ein Laser sein kann, Öffnungen eingebracht, so dass man nach dem Laminieren die zweite Lage durch die Öffnungen sehen kann.

Überraschend wurde nun gefunden, dass es möglich ist die Substratlage bzw. Substratlagen von Verbunden mittels Laserstrahlung gezielt zu zerschneiden, ohne die Folie zu beschädigen.

Die obige Aufgabe wird daher gelöst durch ein Verfahren zur Herstellung von elastischen Verbunden aus mindestens einer Substratlage und mindestens einer Folienlage, die einen Verbund bilden, wobei das Substrat oder die Substrate mittels Laserstrahlung geschnitten werden, sowie auf diese Weise hergestellte Verbunde.

Unter Elastizität wird im Rahmen der vorliegenden Erfindung ein mechanisches Verhalten verstanden, bei dem die Folie bzw. der Verbund zerstörungsfrei in mindestens einer Richtung um mehr als 20 %, insbesondere um mehr als 50 % und ganz besonders bevorzugt um mehr als 100 % gedehnt werden kann. Bei einer Dehnung um 100 % nimmt nach Entfernen der Dehnkraft der Verbund im wesentlichen die Ausgangsform wieder an, d.h. nicht mehr als 30 %, insbesondere 20 % und ganz besonders bevorzugt nicht mehr als 10 % der Verformung bleibt zurück (Restverformung).

Während die Dehnung, die Dehnkraft, bzw. das Dehnverhalten von Verbunden, bestehend aus einer elastischen Folie und einem nicht elastischen Substrat, vor allem durch das Dehnverhalten des Substrates bestimmt wird, wird die Rückverformung bzw. die Restverformung vor allem durch die Art und Qualität der elastischen Folie bestimmt.

Das Dehnverhalten (Dehnkraft) wird mittels Kraft-Dehnungskurven bestimmt, das Rückstellverhalten der Folie wird mittels sogenannter Hysteresekurven ermittelt. Dabei wird die Folie bzw. das Laminat um eine, je nach Anwendungfall, bestimmte Länge gedehnt (z.B. 100 %) und nach einer bestimmten Haltezeit (z.B. 30 sec.) wieder auf den ursprünglichen Zustand entspannt. Anschliessend beginnt mindestens ein weiterer Dehnzyklus. Den dabei zurückgelegten Weg ab dem dann ein Kraftanstieg erfolgt (z.B. F >/= 0,1 N), bezeichnet man als (bleibende) Verformung. Die Messungen erfolgen nach EN ISO 527 mit einer mechanischen Zugprüfmaschine, mit der sowohl der zurückgelegte Weg (Dehnung) als auch die dabei aufgenommene Kraft gemessen und registriert wird. (z.B. Zugmessgerät der Fa. Zwick, Type Z1.0/TH1P mit entsprechender Software).

Insbesondere im Hygienebereich werden elastische Laminate gefordert, die bei 50 % Dehnung eine Kraft von weniger als 4, vorzugsweise weniger als 2 N/cm (5 N/inch) und bei 100 % Dehnung eine Kraft von weniger als 8, vorzugsweise weniger als 4 N/cm (10 N/inch), sowie eine Reissdehnung (Bruchdehnung) von mindestens 400 % und eine Restverformung bei 100 % Dehnung von maximal 30 % aufweisen.

Erfindungsgemäß wird zunächst in an sich bekannter Weise ein Verbund aus Substratlage(n) und Folienlage(n) erzeugt. Es sind verschiedene Techniken bekannt und einsetzbar, z.B. Laminierung mit Klebstoff, Thermobonding und Direktextrusion. Bei den ersten beiden Varianten werden Folie(n) und Substrat(e) zunächst separat hergestellt und dann in einem weiteren Schritt entweder mittels Klebstoff oder durch punktuelles Aufbringen von hohem Druck und hoher Temperatur zu einem Verbund vereinigt. Bei der Direktextrusion wird die Folie, die auch eine mehrlagige Folie sein kann, direkt auf das vorgefertigte Substrat extrudiert.

Bei der vorliegenden Erfindung ist das Verfahren der Extrusionsbeschichtung (Direktextrusion) bevorzugt, wobei mittels Cast-Extrusion eine sich noch im Schmelzezustand befindliche Folie z.B. aus elastischen Rohstoffen in den Walzenspalt (typischerweise einer Stahlwalze und einer Gummiwalze) extrudiert wird. Gleichzeitig wird zwischen Schmelze und Stahlwalze oder zwischen Schmelze und Gummiwalze oder auf beiden Walzen z.B. ein Kardenvlies aus z.B. Polypropylen zugeführt, so dass zwischen noch heisser elastischer Folienschmelze und Vlies ein thermischer Verbund aus elastischer Folie und ein- oder beidseitig aufgebrachtem Vlies entsteht. Sofern zwischen Folienschmelze und entweder Gummiwalze-oder Stahlwalze nur eine Lage Vlies zugeführt wird, erhält man ein Bilaminat aus elastischer Folie und einer Lage Vlies.

Die erfindungsgemäßen Verbunde bestehen aus einer oder mehreren, z.B. zwei, drei, vier, Folien und einem oder mehreren, z.B. zwei drei, vier, Substraten. Bevorzugt sind Verbunde aus einer ein- oder mehrlagigen Folie und einem Substrat auf einer Seite der Folie oder je einem Substrat auf beiden Seiten der Folie. Im letzteren Fall können die Substrate gleich oder verschieden sein.

Die Herstellung der Folien und Substrate sowie die geeigneten Materialien sind dem Fachmann an sich bekannt. Es eignen sich dieselben Substrate und Folien, die auch in den bekannten mechanisch aktivieren Verbunden zum Einsatz kommen.

Als Substrat eignen sich Vliese und Gewirke, die entweder mittels Spinnprozess (Spinnvliese), Kardierprozess (Kardenvliese) oder durch den so genannten "meltblown" Prozess hergestellt werden. Um diese Vliese weiterverarbeiten zu können, werden die losen Fasern durch einzelne Schweißpunkte miteinander verbunden. Neben diesem so genannten Thermobondierprozess kann die Verbindung der einzelnen Fasern auch durch chemischen Verbund oder durch Wasserstrahlen erfolgen. Es ist auch möglich, Substrate aus Fasermischungen einzusetzen. Bevorzugte Substrate sind Polyolefinvliese z.B. Kardenvliese aus Polypropylen oder Spinnvliese aus Polypropylen oder Polyethylen verschiedener Hersteller, wie z.B. das Kardenvlies aus Polypropylen der Type Prosoft der Fa. RKW AG Gronau, mit einem Flächengewicht im Bereich von 15 g/m² bis 35 g/m².

Es ist im Rahmen der Erfindung auch möglich, Substrate einzusetzten, die elastisch sind jedoch für den gewünschten Einsatz noch nicht ausreichend elastisch. In solchen Fällen wird durch das erfindungsgemäße Verfahren eine Steigerung der Elastizität auf das erforderliche bzw. gewünschte Maß erreicht. Insbesondere kann hierbei, wie weiter unten beschrieben werden wird, eine unterschiedliche Elastizität für verschiedene Richtungen eingestellt werden.

Die Folien können mittels Blasextrusion oder Castverfahren hergestellt werden. Besonders bevorzugt werden die Folien direkt auf die Substratlage(n) extrudiert. Die Materialien müssen hierbei so gewählt werden, dass eine ausreichende Haftung erreicht wird, d.h. die Materialien von Folie und Substrat müssen so weit verträglich sein, dass eine Verbindung entsteht. Geeignete Materialien und Materialmischungen sind dem Fachmann bekannt bzw. können anhand einiger Vorversuche festgelegt werden.

Materialien zur Herstellung elastischer Folien sind dem Fachmann bekannt. Elastische Polymere auf Basis von Polyolefinen wie Polyethylen und Polypropylen, Styrol-Butadien-Styrol (SBS), Styrol-Ethylen-Butylen-Styrol (SEBS), Styrol-Isopren-Styrol (SIS) und/oder Mischungen (Kompounds) daraus werden häufig eingesetzt. Die elastische Folie kann z.B. auch aus so genannten elastischen Thermoplasten auf Basis von Metallocen-katalysierten Polyolefinen (Polypropylen (PP), low density Polyethylen (LDPE), lienar low density Polyethylen (LLDPE)) oder Polyurethanen bestehen. Es ist weiterhin denkbar Naturkautschuk zu verwenden, üblicherweise wird davon jedoch wegen möglicher allergischer Reaktionen abgesehen. Ein Verbund mit Substraten (z.B. PP Vliesen) wird beim Einsatz von Naturkautschuk mittels Klebeverbindung erreicht.

Besonders bevorzugt sind ein- und mehrlagige Folien die aus elastischen Polyolefinen (z.B. der Typenreihe Vistamaxx der Fa. ExxonMobile oder der Typenreihe Affinity der Fa. DOW) und/oder Mischungen aus SBS und/oder SEBS Kompounds (z.B. aus der Typenreihe Kraton der Fa. Kraton Polymers oder der Typenreihe Styroflex der Fa. BASF) bestehen. Einige Hersteller bieten fertige, auf den Anforderungen des Kunden abgestimmte elastische Compounds an. (z.B. Fa. Kraigurg TPE GmbH, Fa. Elastoteknik AB oder die Fa. Wittemburg B.V.)

Die Materialien von Substratlage(n) und Folienlage(n) können in an sich bekannter Weise Additive und andere Zusätze enthalten. Beispielhaft sei auf Verarbeitungshilfsmittel, UV- und Wärmestabilisatoren sowie Additive zur Hydrophobierung oder Hydrophilierung verwiesen. Es ist möglich die Substratlage(n) oder Folienlage(n) oder Substrat- und Folienlage(n) einzufärben (pigmentieren) oder zu bedrucken oder beides. Ein Bedrucken kann vor oder nach der Aktivierung mittels Laserstrahlung erfolgen.

Erfindungsgemäß wird der Verbund mittels Laserstrahlung aktiviert. Hierbei wird die Substratlage durch den Laserstrahl geschnitten. Die Strahlung kann entlang gerader, paralleler Linien oder aber entlang gebogener Linien einwirken. Auch Kreuzungen sind möglich. Es ist möglich, den Verbund über seine gesamte Fläche zu aktivieren oder aber nur in Teilbereichen. Die Linien können durchgehend oder unterbrochen sein.

Die Aktivierung mittels Laserstrahl kann sowohl inline, also direkt nach der Laminierung des Verbundes erfolgen, oder aber auch in einem zweiten Arbeitsschritt danach.

Überraschend wurde gefunden, dass es mit der Aktivierung durch Laserstrahlung möglich ist, die Elastizität und Dehnbarkeit von Verbunden zu steuern. Je nach Liniendichte und Linienführung kann sowohl das Ausmaß der Elastizität bzw. der Dehnbarkeit als auch die Richtung bestimmt werden. So führen diagonale Linien, welche ein Rautenmuster bilden, zu einer besonders gleichmäßigen Elastizität. Linien nur in Quer- oder Längsrichtung ergeben Verbunde mit einer hohen Elastizität in Längs- bzw. Querrichtung und einer geringen Dehnbarkeit in der jeweils anderen Richtung. Durch die Wahl von verschieden langen Unterbrechungen lässt sich eine insgesamt höhere oder niedrigere Elastizität und Dehnbarkeit einstellen.

Es ist auch besonders leicht möglich, eine Aktivierung beliebig gestalteter Teilbereiche vorzunehmen. Dies ist bei einer mechanischen Aktivierung mit hohem Aufwand verbunden, weil für jede individuelle Gestaltung die notwendigen Vorrichtungen, insbesondere Walzen, geschaffen werden müssen.

Erfindungsgemäß können komplexe geometrische Schnitte in dem Substrat durchgeführt werden, so dass runde oder Kreuzungsschnitte entstehen. Durch entsprechende Laserfokussierung können die Schnitte entsprechend genau und scharf oder aber auch diffus erfolgen. Auf diese Art und Weise ist es möglich, durch die Wahl der Aktivierungsschnitte, das Substrat derart zu gestalten, dass bei ein und derselben elastischen Folie verschiedene Elastizitäten des Verbundes resultieren.

In einer ersten bevorzugten Ausführungsform wird das Substrat in Längs- oder Querlinien geschnitten. Durch parallele Schnitte in Längsrichtung (Maschinenrichtung), ist ein Verbund erhältlich, das in Querrichtung zur Maschinenrichtung elastisches Verhalten zeigt. Bei dem gleichem Ausgangsmaterial ergeben parallele Schnitte in Querrichtung einen Verbund, der in Längsrichtung elastisches Verhalten aufweist.

In einer zweiten bevorzugten Ausführungsform wird das Substrat entlang solcher Linien geschnitten, dass die Schnitte Muster wie Rauten, Rechtecke oder Quadrate bilden. Durch parallele Schnitte diagonal zur Bahnrichtung entsteht ein geometrisches Muster aus Rauten. Dadurch wurde der Verbund in allen Richtungen, besonders stark in diagonaler Richtung, elastisch.

In einer dritten bevorzugten Ausführungsform wird das Substrat entlang wellen- oder zackenförmiger Linien geschnitten.

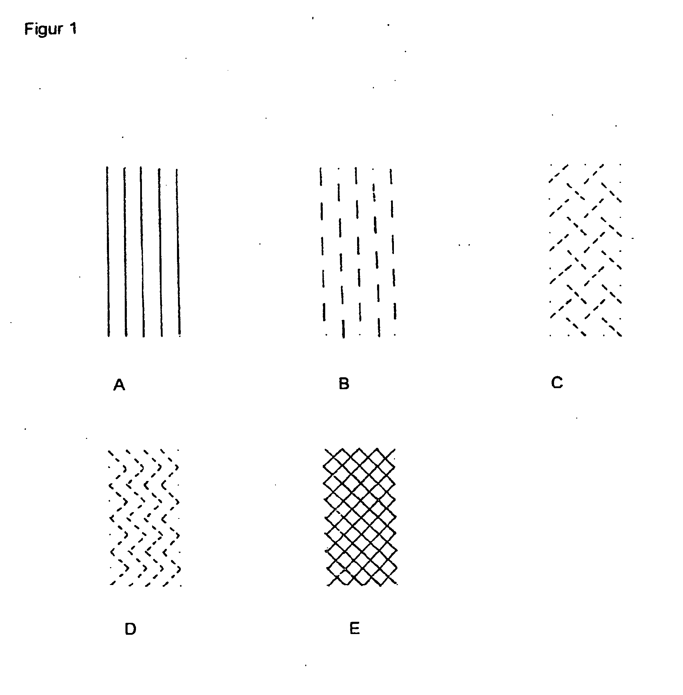

In der Figur 1 sind einige bevorzugte Muster für die Schnitte dargestellt. Bei den Ausführungsformen A und B ergibt sich eine hohe Elastizität in einer Richtung mit einer geringen Elastizität in der anderen. Die Muster C und E führen zu einer gleichmäßigeren Elastizität, während bei Muster D wiederum ein ungleichmäßiger Grad an Elastizität resultiert. In quantitativer Hinsicht ergibt sich für Muster A eine höhere Elastizität als für Muster B, für Muster C eine geringere als für Muster E.

Der Laser ist in seiner Wellenlänge auf die Materialien von Substrat und Folie abzustimmen. Bei Substraten in Form von Polyolefinvliesen hat sich ein CO₂-Laser mit einer Wellenlänge im Bereich von 9,6 bis 10,2 µm als geeignet erwiesen. Die Laserstrahlung wird mittels geeigneter Umlenk- und Fokussiereinrichtungen, wie Spiegel und Linsen, auf den Verbund gelenkt. Der Laserstrahl kann in zwei oder mehr Einzelstrahlen aufgeteilt werden. Es ist zweckmäßig, wenn der Verbund an dem Laser entlang transportiert wird. Je nach gewünschten Linien bleibt der Laserstrahl hierbei ortsfest oder wird ebenfalls bewegt. Zur Schaffung von unterbrochenen Linien kann ein gepulster Laser verwendet werden oder eine entsprechende Optik blendet den Laserstrahl für die gewünschte Zeit aus. Über die Pulsfrequenz bzw. die Ausblendzeiten und/oder die Geschwindigkeit des Verbundtransportes lassen sich Anzahl und Länge der Unterbrechungen leicht einstellen. Die für die Durchtrennung des Substrates nötige Laserleistung hängt vor allem von der Schnittgeschwindigkeit (Bahngeschwindigkeit des Verbundes) und der Anzahl der Schnittlinien im Substrat ab und kann im Einzelfall leicht festgelegt werden.

Der Abstand der Schnittlinien im Substrat kann frei gewählt werden. Durch den Abstand und die Anzahl der Schnittlinien wird das elastische Verhalten des Verbundes ebenso bestimmt, wie durch deren Anordnung (Richtung).

Für die Anwendung im Hygienebereich sollte der Abstand der Schnittlinien zwischen 1 mm und 10 mm, bevorzugt zwischen 2 mm und 5 mm betragen.

Zur Erzeugung spezieller Effekte ist es denkbar, dass ein mittels Laserstrahlen aktiviertes elastische Laminat zusätzlich noch eine mechanische Dehnung (Aktivierung) erhält. Diese mechanische Aktivierung kann sowohl in Richtung der mittels Laser geschnittenen Linien, als auch quer dazu erfolgen. Es ist jedoch bevorzugt, wenn keine mechanische Aktivierung erfolgt, das heißt, die gewünschte Elastizität wird durch das erfindunggemäß Verfahren erreicht, ohne dass eine mechanische Aktivierung notwendig ist.

Die erfindungsgemäßen Verbunde eignen sich als Material zur Herstellung von absorbierenden Einwegartikeln wie Babywindeln, Inkontinenzprodukten und Damenhygienartikeln. Hierbei ist es besonders vorteilhaft, dass den Verbunden eine gezielt steuerbare Elastizität auch in Teilbereichen verliehen werden kann. So ist es z.B. möglich die Außenlage von Babywindeln (Backsheet) mit den Bündchen, dem Bundbereich und den Verschlussbereichen einstückig auszubilden und nur im Bundbereich eine mäßige und im seitlichen Verschlussbereich sowie bei den Bündchen eine starke Elastizität zu erzeugen. Bisher wurden insbesondere die Verschlussbereiche, der Bundbereich und die Beinbündchen häufig aus einem separaten, hochelastischen Material gesondert gefertigt und mussten dann aufwändig mit der restlichen Außenlage verbunden werden.

Eine weitere Anwendung ist im Bereich von Einwegbekleidung gegegeben, z.B. als Material für Schutzkleidung im Medizinbereich, wie Kittel, Hauben etc.

Die Erfindung soll anhand der folgenden Beispiele näher erläutert werden, ohne jedoch auf die konkret beschriebenen Ausführungsformen beschränkt zu sein. Im Rahmen der vorliegenden Anmeldung beziehen sich alle Angaben von % und Teilen, wenn nichts anderes angegeben ist, auf das Gewicht.

### Beispiel 1:

Auf einer Cast Extrusionslinie wurde eine Mischung, bestehend aus einem elastischem Polyolefin der Fa. ExxonMobile, einem elastischen Polyolefin der Fa. Dow, einem SEBS-Kompound der Fa. Kraton Polymers und einem Weiß-Pigment der Fa. Clariant, mittels Extrusionsbeschichtung auf ein Kardenvlies der Fa. RKW AG Gronau laminiert, wobei das Kardenvlies im Walzenspalt einer Stahl- und Gummiwalze jeweils beidseitig mit der sich noch in der Schmelze befindlichen Folie verbunden wurde. Dadurch entstand ein 3 schichtiger Verbund aus einer elastischen Folie und beidseitig aufgebrachtem nicht elastischem Vlies. Der Verbund war nicht elastisch und nur mit - für Hygieneverhälnisse - hohem Kraftaufwand dehnbar. Die Folie hatte ein Flächengewicht von 60 g/m², eine Vlieslage hatte ein Flächengewicht von 27 g/m², so dass der Verbund ein gesamtes Flächengewicht von 114 g/m² hatte.

Dieser Verbund wurde dann einer Laser Behandlung unterzogen, indem das Vlies auf beiden Seiten mittels Laserstrahl definiert durchtrennt wurde. Als Laser kam ein CO₂ Laser mit einer Wellenlänge von 9,6 - 10,2 µm zu Einsatz. Die Durchtrennung der beiden außen liegenden Vliese erfolgte symmetrisch mit der elastischen Folie als Symmetrieachse. Bei diesem Versuch wurden zwei verschiedene Laser-Schnittmuster programmiert:
1. unterbrochene parallele Linien in Maschinenlaufrichtung (Muster B in Figur 1)
2. unterbrochene parallele "zick-zack" Linien in Maschinenlaufrichtung (Muster D in Figur 1)

Folgende Kraft - Dehnungswerte (EN ISO 527) wurden für die drei Verbunde bestimmt (Einspannlänge: 50mm; Prüfgeschwindigkeit: 300mm/min):

| Eigenschaft | Verbund ungeschnitten | Verbund geschnitten Muster B | Verbund geschnitten Muster D |
|---|---|---|---|
| Kraft bei 50 % Dehnung [N/cm] | 5,2 | 1,4 | 1,3 |
| Kraft bei 100 % Dehnung [N/cm] | 6,1 | 3,7 | 3,6 |
| Kraft bei 150 % Dehnung [N/cm] | 6,5 | 5,0 | 4,8 |
| Reissfestigkeit [N/cm] | 7,0 | 8,7 | 9,8 |
| Bruchdehnung [%] | 320 | 500 | 550 |
| Restverformung bei 100% Dehnung [%] | 10,8 | 4,8 | 3,5 |

Aus den Ergebnissen ist deutlich zu erkennen, dass durch eine Laseraktivierung das Dehnverhalten und somit die elastischen Eigenschaften eines Verbundes, bestehend aus elastischer Folie und Vlies positiv beeinflusst werden und sich durch die Wahl der Laserschnitte quantitativ einstellen lassen.

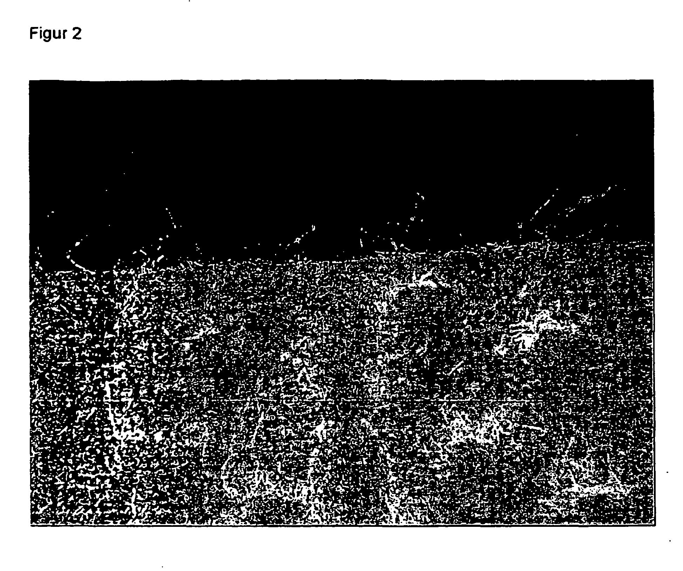

Figur 2 zeigt eine mikroskopische Aufnahme eines mittels Laser aktivierten Vlies-Folienverbundes. Man erkennt gut die Thermobondierpunkte in dem Vlies sowie den mittels Laserstrahlung erzeugten Schnitt in der Mitte.

## Patentansprüche

1. Verfahren zur Herstellung elastischer Verbunde aus mindestens einer Substratlage und mindestens einer Folie umfassend die Schritte
a) Bereitstellen von einer oder mehreren Substratlagen
b) Verbinden der Substratlage(n) mit einer oder mehreren elastischen Folienlagen, die zerstörungsfrei in mindestens einer Richtung um mehr als 20 % gedehnt werden können, wobei bei einer Dehnung um 100 %_nach Entfernen der Dehnkraft nicht mehr als 30 % der Verformung zurückbleibt,
c) Durchschneiden der Substratlage(n) mittels Laserstrahlung.

2. Verfahren gemäß Anspruch 1, **dadurch gekennzeichnet, dass** die Substratlage(n) mittels Kleberlaminierung oder Thermobonding mit einer oder mehreren elastischen Folienlagen verbunden werden.

3. Verfahren gemäß Anspruch 1, **dadurch gekennzeichnet, dass** die elastische(n) Folienlage(n) direkt auf die Substratlage(n) extrudiert werden.

4. Verfahren gemäß mindestens einem der Ansprüche 1 bis 3, **dadurch gekennzeichnet, dass** die Laserstrahlung unterbrochene Linien in die Substratlage(n) schneidet.

5. Verfahren gemäß mindestens einem der Ansprüche 1 bis 3, **dadurch gekennzeichnet, dass** die Laserstrahlung durchgehende Linien in die Substratlage(n) schneidet.

6. Verfahren gemäß mindestens einem der Ansprüche 1 bis 5, **dadurch gekennzeichnet, dass** die Substratlage(n) entlang gerader, paralleler Linien durchschnitten werden.

7. Verfahren gemäß mindestens einem der Ansprüche 1 bis 5, **dadurch gekennzeichnet, dass** die Substratlage(n) entlang sich kreuzender oder geschlossener Linien durchschnitten werden.

8. Verfahren gemäß Anspruch 7, **dadurch gekennzeichnet, dass** die Linien geometrische Muster wie Rauten, Rechtecke oder Quadrate bilden.

9. Verfahren gemäß Anspruch 7, **dadurch gekennzeichnet, dass** die Linien Muster wie Kreise, Ellipsen oder Kreuze bilden.

10. Verfahren gemäß mindestens einem der Ansprüche 1 bis 5, **dadurch gekennzeichnet, dass** die Substratlage(n) entlang gebogener, wellenförmiger oder zick-zack-förmiger Linien geschnitten werden.

11. Verfahren gemäß mindestens einem der Ansprüche 1 bis 10, **dadurch gekennzeichnet, dass** die Substratlage(n) mit einem CO₂-Laser geschnitten werden.

12. Elastischer Verbund aus mindestens einer Substratlage und mindestens einer Folienlage, wobei eine oder mehrere nicht-elastische Substratlage(n) mit einer oder mehreren elastischen Folienlagen, die zerstörungsfrei in mindestens einer Richtung um mehr als 20 % gedehnt werden können, wobei bei einer Deheneug um 100 % nach Entfernen der Dehnkraft nicht mehr als 30 % der Verformung zurückbleibt, verbunden sind **dadurch gekennzeichnet, dass** die Substratlage mittels Laserstrahlung eingebrachte Schnitte aufweist, wobei die Schnittdichte und Anordnung eine vorbestimmte Elastizität in mindestens einer Richtung gewährleistet.

13. Elastischer Verbund gemäß Anspruch 12, **dadurch gekennzeichnet, dass** die Substratlage(n) Vliese oder Gewirke aus Polyolefinen sind.

14. Elastischer Verbund gemäß Anspruch 12 oder 13, **dadurch gekennzeichnet, dass** die Folienlage(n) aus Polyolefinen, Styrol-Butadien-Styrol (SBS), Styrol-Ethylen-Butylen-Styrol (SEBS), Styrol-Isopren-Styrol (SIS) und/oder Mischungen daraus bestehen.

15. Elastischer Verbund gemäß mindestens einem der Ansprüche 12 bis 14, **dadurch gekennzeichnet, dass** die Bruchdehnung mindestens 400 % beträgt.

16. Elastischer Verbund gemäß mindestens einem der Ansprüche 12 bis 15, **dadurch gekennzeichnet, dass** die Restverformung nach einer Dehnung um 100 % höchstens 30 % beträgt.

17. Elastischer Verbund gemäß mindestens einem der Ansprüche 12 bis 16, **dadurch gekennzeichnet, dass** der Verbund aus einer Folienlage und einer Substratlage besteht.

18. Elastischer Verbund gemäß mindestens einem der Ansprüche 12 bis 16, **dadurch gekennzeichnet, dass** der Verbund aus einer Folienlage und je einer Substratlage, die gleich oder verschieden sein können, auf jeder Seite der Folie besteht.

19. Elastischer Verbund gemäß mindestens einem der Ansprüche 12 bis 18, **dadurch gekennzeichnet, dass** die Folienlage eine mehrschichtige Folie ist.

20. Elastischer Verbund gemäß mindestens einem der Ansprüche 12 bis 19, **dadurch gekennzeichnet, dass** dieser zusätzlich einer Dehnung unterzogen wird.

## Claims

1. A method for manufacturing elastic composite material comprising at least one substrate layer and at least one film, comprising the steps
a) preparing one or more substrate layers
b) joining the substrate layer(s) to one or more elastic film layers, which can be non-destructively elongated by more than 20% in at least one direction, wherein, if elongated by 100%, no more than 30% of the deformation remains after the expansion force is removed,
c) cutting the substrate layer(s) using laser radiation.

2. The method according to claim 1, **characterized in that** the substrate layer(s) are joined to one or more elastic film layers using adhesive lamination or thermobonding.

3. The method according to claim 1, **characterized in that** the elastic film layer(s) are extruded directly onto the substrate layer(s).

4. The method according to at least one of the claims 1 to 3, **characterized in that** the laser radiation cuts interrupted lines into the substrate layer(s).

5. The method according to at least one of the claims 1 to 3, **characterized in that** the laser radiation cuts continuous lines into the substrate layer(s).

6. The method according to at least one of the claims 1 to 5, **characterized in that** the substrate layer(s) are cut along straight, parallel lines.

7. The method according to at least one of the claims 1 to 5, **characterized in that** the substrate layer (s) are cut along intersecting or closed lines.

8. The method according to claim 7, **characterized in that** the lines form geometric patterns such as diamonds, rectangles or squares.

9. The method according to claim 7, **characterized in that** the lines form patterns such as circles, ellipses or crosses.

10. The method according to at least one of the claims 1 to 5, **characterized in that** the substrate layer(s) are cut along curved, wave-shaped or zigzag lines.

11. The method according to at least one of the claims 1 to 10, **characterized in that** the substrate layer(s) are cut using a CO₂ laser.

12. An elastic composite material comprising at least one substrate layer and at least one film layer, wherein one or more non-elastic substrate layer(s) are joined to one or more elastic film layers, which can be non-destructively elongated by more than 20% in at least one direction, wherein, if elongated by 100%, no more than 30% of the deformation remains after the expansion force is removed, **characterized in that** the substrate layer has cuts formed using laser radiation, wherein the density of the cuts and the arrangement ensures a predetermined elasticity in at least one direction.

13. The elastic composite material according to claim 12, **characterized in that** the substrate layer(s) are nonwovens or knitted fabric made of polyolefins.

14. The elastic composite material according to claim 12 or 13, **characterized in that** the film layer(s) are made of polyolefins, styrene-butadiene-styrene (SBS), styrene-ethylene-butylene-styrene (SEBS), styrene-isoprene-styrene (SIS) and/or mixtures thereof.

15. The elastic composite material according to at least one of the claims 12 to 14, **characterized in that** the elongation at break is at least 400%.

16. The elastic composite material according to at least one of the claims 12 to 15, **characterized in that** the residual deformation after elongation by 100% is at most 30%.

17. The elastic composite material according to at least one of the claims 12 to 16, **characterized in that** the composite material comprises a film layer and a substrate layer.

18. The elastic composite material according to at least one of the claims 12 to 16, **characterized in that** the composite material comprises one film layer and a substrate layer on each side of the film, wherein the substrate layers can be the same or different.

19. The elastic composite material according to at least one of the claims 12 to 18, **characterized in that** the film layer is a multilayer film.

20. The elastic composite material according to at least one of the claims 12 to 19, **characterized in that** it is additionally subjected to expansion.

## Revendications

1. Procédé de fabrication de composites élastiques formés d'au moins une couche de substrat et d'au moins une feuille, comprenant les étapes:
a) fournir une ou plusieurs couches de substrats,
b) assembler la ou les couches de substrats avec une ou plusieurs couches de feuilles élastiques pouvant s'allonger sans destruction de plus de 20 % dans au moins une direction, sachant que pour un allongement de 100 %, après suppression de la force d'allongement, la déformation permanente ne dépasse pas 30 %,
c) découper la ou les couches de substrats au moyen d'un rayonnement laser.

2. Procédé selon la revendication 1, **caractérisé en ce que** la ou les couches de substrats sont assemblées avec une ou plusieurs couches de feuilles élastiques au moyen d'un contre-collage ou thermobonding.

3. Procédé selon la revendication 1, **caractérisé en ce que** la ou les couches de feuilles élastiques sont extrudées directement sur la ou les couches de substrats.

4. Procédé selon au moins l'une des revendications 1 à 3, **caractérisé en ce que** le rayonnement laser coupe des lignes interrompues dans la ou les couches de substrats.

5. Procédé selon au moins l'une des revendications 1 à 3, **caractérisé en ce que** le rayonnement laser coupe des lignes ininterrompues dans la ou les couches de substrats.

6. Procédé selon au moins l'une des revendications 1 à 5, **caractérisé en ce que** la ou les couches de substrats sont découpées le long de lignes droites parallèles.

7. Procédé selon au moins l'une des revendications 1 à 5, **caractérisé en ce que** la ou les couches de substrats sont découpées le long de lignes croisées ou fermées.

8. Procédé selon la revendication 7, **caractérisé en ce que** les lignes forment des motifs géométriques tels que des losanges, des rectangles ou des carrés.

9. Procédé selon la revendication 7, **caractérisé en ce que** les lignes forment des motifs tels que des cercles, des ellipses ou des croix.

10. Procédé selon au moins l'une des revendications 1 à 5, **caractérisé en ce que** la ou les couches de substrats sont découpées le long de lignes courbes, ondulées ou zigzaguées.

11. Procédé selon au moins l'une des revendications 1 à 10, **caractérisé en ce que** la ou les couches de substrats sont découpées avec un laser au CO₂.

12. Composite élastique formé d'au moins une couche de substrat et d'au moins une couche de feuille, dans lequel une ou plusieurs couches de substrats non élastiques sont assemblées avec une ou plusieurs couches de feuilles élastiques pouvant s'allonger sans destruction de plus de 20 % dans au moins une direction, sachant que pour un allongement de 100 %, après suppression de la force d'allongement, la déformation permanente ne dépasse pas 30 %, **caractérisé en ce que** la couche de substrat comporte des coupes réalisées au moyen d'un rayonnement laser, la densité des coupes et la disposition garantissant une élasticité prédéterminée dans au moins une direction.

13. Composite élastique selon la revendication 12, **caractérisé en ce que** la ou les couches de substrats sont des non-tissés ou des maillages en polyoléfines.

14. Composite élastique selon la revendication 12 ou 13, **caractérisé en ce que** la ou les couches de feuilles sont constituées de polyoléfines, de styrène-butadiène-styrène (SBS), de styrène-éthylène-butylène-styrène (SEBS), de styrène-isoprène-styrène (SIS) et/ou de mélanges de ceux-ci.

15. Composite élastique selon au moins l'une des revendications 12 à 14, **caractérisé en ce que** l'allongement à la rupture est au moins de 400 %.

16. Composite élastique selon au moins l'une des revendications 12 à 15, **caractérisé en ce que** la déformation résiduelle, après un allongement de 100 %, ne dépasse pas 30 %.

17. Composite élastique selon au moins l'une des revendications 12 à 16, **caractérisé en ce que** le composite est constitué d'une couche de feuille et d'une couche de substrat.

18. Composite élastique selon au moins l'une des revendications 12 à 16, **caractérisé en ce que** le composite est constitué d'une couche de feuille et de respectivement une couche de substrat, qui peuvent être identiques ou différentes, de chaque côté de la feuille.

19. Composite élastique selon au moins l'une des revendications 12 à 18, **caractérisé en ce que** la couche de feuille est une feuille multicouche.

20. Composite élastique selon au moins l'une des revendications 12 à 19, **caractérisé en ce que** celui-ci est additionnellement soumis à un allongement.
